(19) Europäisches Patentamt

European Patent Office

Office européen des brevets

(11) **EP 2 316 515 B1**

(12) **EUROPEAN PATENT SPECIFICATION**

(45) Date of publication and mention
of the grant of the patent:
**17.07.2019  Bulletin 2019/29**

(21) Application number: **09174294.0**

(22) Date of filing: **28.10.2009**

(51) Int Cl.:
*A61M 16/01* *(2006.01)*

(54) **Arrangement for controlling narcotic effect value of breathing gas**

Anordnung zur Steuerung des Narkoseauswirkungswertes von Atemgas

Agencement pour contrôler la valeur d'effet narcotique de gaz respirable

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**04.05.2011  Bulletin 2011/18**

(73) Proprietor: **General Electric Company
Schenectady, NY 12345 (US)**

(72) Inventors:
• **Heinonen, Erkki
00750 Helsinki (FI)**

• **Häggblom, Tom
01520 Vantaa (FI)**

(74) Representative: **Fennell, Gareth Charles et al
Kilburn & Strode LLP
Lacon London
84 Theobalds Road
London WC1X 8NL (GB)**

(56) References cited:
**WO-A2-2006/094172      US-A1- 2009 090 359
US-A1- 2009 124 867**

**Description**

BACKGROUND OF THE INVENTION

This disclosure relates generally to a method and arrangement for controlling a narcotic effect value of a mixture of at least two narcotic agents of a breathing gas.

In order to perform surgery the patient must be anesthetized. This incorporates hypnosis and pain prevention and is achieved with anesthetizing drugs. The most common drugs used for the purpose are inhalation anesthetics. These are either gases ($N_2O$) or volatile liquids (inhalation agents). Before delivery to patient breathing the inhalation agents are converted to gaseous form in anesthesia vaporizers. The anesthesia delivery systems may comprise multitude of vaporizers for different agents, or the vaporizer is easily exchangeable to another. The delivery systems are designed to allow delivery of one agent at a time, but for various reasons a need to change the agent from one to another may emerge. Such reasons are e.g. better acceptance of one agent when patient is still awake during anesthesia induction and faster recovery from anesthesia or less post-anesthesianausea of another. Examples of such systems are disclosed in WO 2006/094172.

Once vaporized, the anesthetic agent is delivered to anesthesia breathing circuit for delivery to patient. The anesthesia breathing system allows re-breathing of the patient exhaled breathing gas. This re-breathing is used to preserve patient expired expensive and environmental-hostile anesthesia agent vapors to reduce the agent consumption.

The re-breathing circuit comprises of inspiration- and expiration limbs, Y-piece, CO2 absorber to refresh the exhalation gas for re-breathing, and ventilator tubing. Other breathing gas volumes are the ventilator gas isolation system and patient lungs. The total gas volume of the system may be up to 7 liters.

The breathing gas meets the blood circulation in the lungs. There the agent dissolves into the blood that transports the agent to further body. From the blood the agent dissolves further to various tissues including the site of anesthetic effect in brains. Each of the agents has their own threshold brain concentration to be achieved in order to make the patient anesthetized. The required concentration is also patient specific, but for each agent a mean alveolar concentration (MAC) has been determined. This describes the concentration of the vapor measured in percentage at 101.3 kPa ambient pressure preventing a patient movement under a surgical stimuli of a skin incision in 50% of patients (Edmond I Eger II: Age, Minimum Alveolar Anesthetic Concentration, and Minimum Alveolar Anesthetic Concentration-Awake. Anesth Analg. 2001; 93:947-53). To reach a higher confidence level for a proper anesthesia the patients are usually given the agent corresponding 1.2-1.3 MAC. Concentration corresponding to 1 MAC is 2.1% for sevoflurane, 6,0% for desflurane, 1,1% for isoflurane, 0,76% for halothane, 1.7% for enflurane, and 101% for nitrous oxide ($N_2O$). These values are for patients of 40 year old. The values are decreasing with the age.

MAC may be determined also for other indications, MAC-Awake is an example of this determining the concentration suppressing a response on commands in 50% of patients. For narcotics the MAC-Awake is of the order of 1/3 of the MAC.

At steady state anesthesia the breathing circuit volumes and the patient tissues are filled to this required concentration. Patient anesthesia status is measured as end-tidal concentration of the agent determined as the gas concentration exhaled by the patient at end of expiration. At steady state this equals the effect site concentration and corresponds with patient depth of anesthesia. Solubility of the agent to the body tissues and the required concentration determines the amount of the agent present in the system and patient tissues. Concentration in large tissue volumes and the amount of drug in the patient increases slowly before the steady state is achieved.

As agent induction, agent clearance from the body occurs as well through the lungs: When the breathing gas ventilating the lungs have the agent concentration low compared to alveols of the lungs, the agent concentration of the alveol will decrease. Higher anesthetic agent vapor pressure of blood drives for diffusion of the agent from blood to alveoli decreasing the blood vapor pressure. Similarly the reduced blood vapor pressure allows clearance from tissues having higher vapor pressure. Depending on agent, its solubility to tissues, and size of the tissues determines the clearance of the body from the anesthesia agents.

The effect of the anesthesia agents is linear and additive. Thus, if the subject gets properly anesthetized with the narcotic effect value such as 1 MAC, this may be achieved e.g. with delivery of 0.5 MAC of sevoflurane (= 0.5 x 2.1% = 1.05%) and 0.5 MAC of $N_2O$ (=0.5 x 101% = 50.5%). Normally the agent mixtures occur when delivering $N_2O$ as fresh gas and completing its narcotic effect with some volatile agent. Due to the additive nature of the anesthetizing effect of the different agents, if changing the concentration of one agent, in order to preserve the total effect, changing the other agent as well must compensate the change.

The patient breathing gas is a mixture of oxygen ($O_2$), balance gas $N_2O$ or $N_2$, and the volatile agent. Patient concentration for $O_2$ varies normally between 25-35 volume %, but may sometimes increase over 80%. The patient agent concentration depends on the MAC value and varies from 1 to 10 volume %. The rest of the mixture is balance gas. The balance gas concentration depends on the $O_2$ concentration required. If that needs to be changed for any therapeutic reasons and $N_2O$, is used as a balance gas, the anesthetizing effect (MAC reading) of the $N_2O$ becomes changed as well. Therefore in order to maintain anesthesia, delivery of volatile anesthetics must be adjusted as well.

The additive behavior of the agents need to be taken into consideration especially if changing from one volatile agent to another during anesthesia: Before switchover the old agent corresponds to one MAC maintaining proper anesthesia, and after switchover the new agent does the same. During the switchover the sum of the old and new agent should sum up to the desired MAC. This is difficult in practice since the clearance of the old agent depends on various aspects, as the patient size, agent, and the agent saturation of the tissues, and wash-in of the new agent should be synchronized with the clearance of the old concentration that is measured as the patient exhalation gas concentration.

Inhalation anesthesia delivery control systems may enclose closed loop control of vaporizer in order to match the measured patient end-tidal anesthesia agent concentration to match with user given target. Such system helps the anesthesiologist to reach and maintain determined anesthesia level throughout various changes during anesthesia. Such control systems do however not solve the whole problem when the anesthesia is delivered as a sum of various anesthetizing agents: The clinician needs still titrate gradually the end-tidal target of the delivered volatile agent as a response of clearance of the previous agent or as a response to $N_2O$ concentration changes

BRIEF DESCRIPTION OF THE INVENTION

The above-mentioned shortcomings, disadvantages and problems are addressed herein which will be understood by reading and understanding the following specification.

In an embodiment, a method for controlling a narcotic effect value of a mixture of at least two narcotic agents of a breathing gas includes setting a target value for a desired narcotic effect of narcotic agents and measuring a breathing gas concentration of each narcotic agent of the breathing gas. The method for controlling a narcotic effect value of a mixture of at least two narcotic agents of a breathing gas also includes converting measured breathing gas concentration of each narcotic agent to a narcotic effect value of the breathing gas and calculating a total narcotic effect value of the mixture of the at least two narcotic agents. The method for controlling a narcotic effect value of a mixture of at least two narcotic agents of a breathing gas further includes comparing the total narcotic effect value with the target value and determining whether or not to change the breathing gas concentration to meet the target value.

In another embodiment, an arrangement for controlling a narcotic effect value of a mixture of at least two narcotic agents of a breathing gas includes a gas delivery unit for supplying a breathing gas including a mixture of at least two narcotic agents for a respiration, the gas delivery unit comprising at least one narcotic agent supply. The arrangement for controlling a narcotic effect value of a mixture of at least two narcotic agents of a breathing gas also includes a gas analyzer for measuring a breathing gas concentration of each narcotic agent of the breathing gas and a user interface for setting a target value for a desired narcotic effect of narcotic agents. The arrangement for controlling a narcotic effect value of a mixture of at least two narcotic agents of a breathing gas further includes a controller for converting the measured concentration of each narcotic agent to a narcotic effect value of the breathing gas, for calculating a total narcotic effect value of the mixture of the at least two narcotic agents, for comparing the total narcotic effect value with the target value and for determining whether or not to change the breathing gas concentration to meet the target value.

In yet another embodiment an arrangement for controlling a narcotic effect value of a mixture of at least two narcotic agents of a breathing gas includes a ventilator configured to control respiratory movements and a gas delivery unit for supplying a breathing gas including a mixture of at least two narcotic agents for a respiration, the gas delivery unit comprising at least one narcotic agent supply. The arrangement for controlling a narcotic effect value of a mixture of at least two narcotic agents of a breathing gas also includes a breathing circuit for conducting an expiration gas flow to the ventilator and for conducting the fresh gas flow from the gas delivery unit for the respiration and for conducting the ventilator gas flow for the inspiration. The arrangement for controlling a narcotic effect value of a mixture of at least two narcotic agents of a breathing gas further includes a gas analyzer for measuring a breathing gas concentration of each narcotic agent of the breathing gas and a user interface for setting a target value for a desired narcotic effect of narcotic agents. The arrangement for controlling a narcotic effect value of a mixture of at least two narcotic agents of a breathing gas also includes a controller for converting the measured concentration of each narcotic agent to a narcotic effect value of the breathing gas, for calculating a total narcotic effect value of the mixture of the at least two narcotic agents, for comparing the total narcotic effect value with the target value and for determining whether or not to change the breathing gas concentration to meet the target value.

Various other features, objects, and advantages of the invention will be made apparent to those skilled in art from the accompanying drawings and detailed description thereof.

BRIEF DESCRIPTION OF THE DRAWINGS

Figure 1 is a schematic view of an arrangement in accordance with an embodiment; and

Figure 2 is a block diagram illustrating a method in accordance with an embodiment.

DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

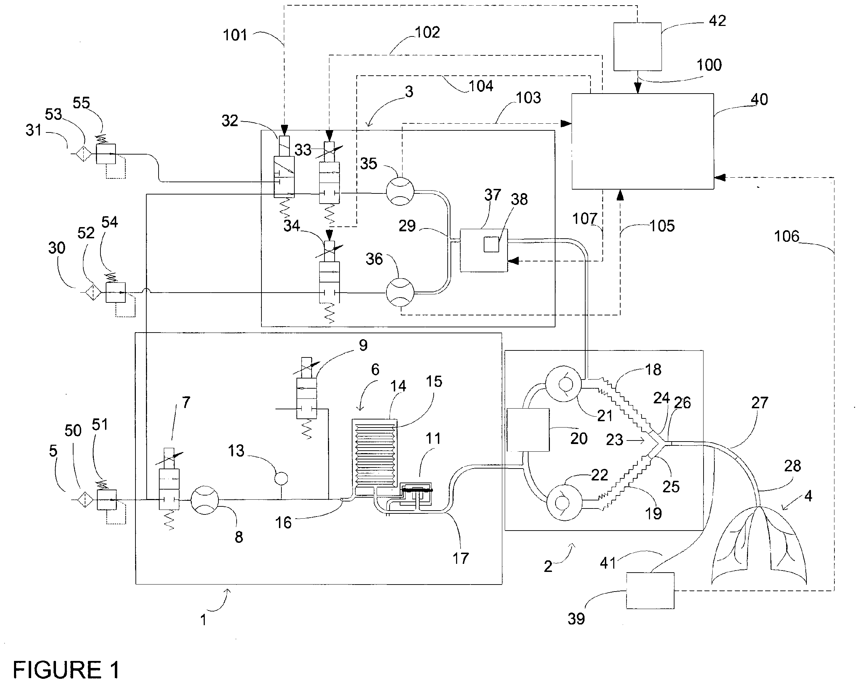

Figure 1 shows an arrangement for controlling a narcotic effect value of a mixture of at least two narcotic agents of a breathing gas. Also a method for controlling a narcotic effect value of a mixture of at least two narcotic agents of a breathing gas is disclosed. The arrangement may comprise a gas delivery unit 3, a gas analyzer 39, a user interface 42 and a controller 40 for controlling the narcotic affect value. The arrangement also may comprise a ventilator 1 and a breathing circuit 2 connecting lungs of a subject 4 with the gas delivery unit 3 and ventilator 1 to exchange the gas in the lungs. The subject 4 is connected to the breathing circuit 2 by means of an endotracheal tube 28.

In Figure 1 the ventilator 1 is connected to a gas supply 5, which is typically pressurized air or sometimes also oxygen. The gas supply 5 is operably connected to filtering 50 and pressure regulation 51. The ventilator 1 may comprise a reciprocating unit 6 for compressing gas towards lungs of the subject to facilitate the inspiration, a flow control valve 7 to control the inspired gas flow from the gas supply 5 towards the reciprocating unit 6, a flow sensor 8 such as a driving gas inspiration flow sensor for measuring a ventilator gas flow added for the inspiration, which flow sensor typically locates between the flow control valve 7 and the reciprocating unit 6. Further the ventilator 1 may comprise an expiration valve 9 used to control the expired gas flow rate releasing the gas of the ventilator 1 when the subject 4 is expiring. The ventilator 1 can also comprise a scavenging valve 11 allowing an extra expired gas of the subject 4 to leave the breathing circuit 2. Very often the ventilator 1 also comprises a ventilator pressure sensor 13 to measure a pressure of the ventilator gas upstream the reciprocating unit 6 and the ventilator 1 may be equipped with a gas supply selection (not shown) that can be used to switch the ventilator gas flow for the inspiration either manually or automatically e.g. in case the used gas gets un-pressurized. The reciprocating unit 6 shown in Figure 1 comprises a bottle 14 and a bellows 15 within the bottle 14 for controlling respiratory movements of the subject's lungs.

When ventilating the subject, the expiration valve 9 is closed and the flow control valve 7 is opened for the inspiration flow. This flow fills the bottle 14 making the bellows 15 to push down pushing the gas within the bellows further towards the subject 4. During the expiration the flow control valve 7 is closed and the expiration valve 9 is opened to control the expiration flow and pressure. The gas pressurized in the bottle 14 is released allowing the gas from lungs to fill the bellows 15 up. When the bellows 15 is filled, it hits the top of the bottle 14, and the further gas flow into the system increases the pressure within the bellows 15. When this pressure exceeds the bottle pressure, the scavenging valve 11 will open allowing the further gas flow to scavenging valve 11.

The ventilator 1 is operably connected to the breathing circuit 2 such as a re-breathing circuit by means of a ventilator tube 17 for both inspired and expired gas flows. The breathing circuit 2 comprises an inspiration tube 18 for inspired gas, an expiration tube 19 for expired gas, a $CO_2$ remover 20 such as $CO_2$ absorber to remove or absorb carbon dioxide from the exhaled gas coming from the subject 4, a first one way valve 21 to allow an inspiration through the inspiration tube 18, a second one way valve 22 to allow an expiration through the expiration tube 19, a branching unit 23 such as a Y-piece having at least three limbs, one of them being an inhalation limb 24 for inspired gas, a second one being an expiration limb 25 for expired gas, a third one being a combined inspiration and expiration limb 26 for both inspired and expired gases. The inhalation limb 24 is connectable to the inspiration tube 18 and the expiration limb 25 is connectable to the expiration tube 19. The combined inspiration and expiration limb 26 of the branching unit 23 may be connectable by means of a patient tube 27 to the endotracheal tube 28 allowing the gas exchange with airways of the subject 4.

The inspiration gas flows from the reciprocating unit 6 through the ventilator tube 17, the $CO_2$ remover 20 and the inspiration tube 18 of the breathing circuit 2 to the branching unit 23 and further through the patient tube 27 and the endotracheal tube 28 to the lungs of the subject 4. The second one-way valve 22 on the expiratory tube 19 guides the gas flow direction to the inspiration tube 18 by closing the flow from the ventilator tube 17 through the expiration tube 19. Increasing the gas volume within the lungs increases the lung pressure due to the lung compliance. Once the inspiration stops and the expiration begins the expiration valve 9 opens relieving the bottle 14 pressure, the lung compliance pushes the alveolar gas through the endotracheal tube 28 and the patient tube 27 to the branching unit 23 and further through the expiration tube 19 and the ventilator tube 17 to fill the bellows 15.

The gas delivery unit 3 for delivering a fresh gas is operably connected to the breathing circuit 2. The gas delivery unit 3 is used to form the subject breathing gas. One or more gas supplies 5, 30, 31 is connected to the gas delivery unit 3. The gas supplies 30 and 31 are just as the gas supply 5 operably connected to respective filterings 52, 53 and pressure regulations 54, 55. The gas supply 5 is for the air as described above including a balance gas such as nitrogen, the gas supply 30 is for oxygen and the gas supply 31 is for an alternate balance gas, which is typically nitrous oxide. Nitrous oxide is also a narcotic agent. The gas delivery unit comprises a selector valve 32 to select either the gas supply 31 for nitrous oxide or the gas supply 5 for air, a flow regulating valve 33 for adjusting a balance gas flow, a flow regulating valve 34 for adjusting oxygen flow and an at least one narcotic agent supply 37 such as a vaporizer for supplying a narcotic agent such as an anesthetic agent to anesthetize the subject 4. The gas delivery unit 3 also comprises flow sensors 35, 36 for measuring the individual fresh gas flows coming from flow regulating valves 33, 34 and which flows may be added into the breathing circuit 2 for respiration. The flow sensor 35 downstream the flow regulating valve 33 is adapted to measure the balance gas flow as a fresh gas, the flow sensor 36 downstream the flow regulating valve 34 may be adapted to measure oxygen flow as a fresh gas.

Using the flow sensor measurement information obtained from the flow sensors 35, 36 the controller 40 regulates the flow regulating valves 33 and 34 to deliver the required gas flows. After flow measurements the individual gas flows are usually merged to a gas mixture at a connection 29. The mixture may then be further guided to the narcotic agent supply 37 for adding the narcotic inhalation agent into the mixture as shown in Figure 1. There may be one or more narcotic agent supplies, but according to the existing device requirements only one narcotic agent supply is allowed to be active for the vaporization at a time. To adjust the narcotic agent vaporized by means of the narcotic agent supply 37 an actuator 38 is needed. Especially in the closed loop narcotic agent control the actuator 38 can be used for automatic control of the narcotic agent delivery by means of the controller 40.The narcotic agent can also be injected directly into the breathing circuit 2 in a liquid or vaporized form by means of the narcotic agent supply 37 when it will be vaporized to the gas in the circuit or as a vapor.

The gas analyzer 39 is enabled to measure the breathing gas or exhalation gas concentrations such as the end-tidal concentrations of all narcotic agents contributing to the narcotic effect value of the subject. This includes typically ability to measure $N_2O$ concentration and mixture of two narcotic agents. The gas analyzer 39 may also identify narcotic agents in case they are not known. The gas analyzer 39 of Figure 1 is a side-stream analyzer equipped with a mechanism that takes a sample of the breathing gas through a sampling line 41 for analysis within the gas analyzer. This sample gas flow is 50-250 mL/min depending on the analyzer. Alternatively the gas analyzer 39 could be a mainstream type connected directly to the breathing circuit 2 or the patient tube 27 without any sampling line 41. In either case gas analyzers are nowadays typically based on infrared absorption technique.

The controller 40 for controlling the narcotic agent value includes a processing unit (not shown in the Figure) to receive the information indicative of the measured breathing gas concentration of the narcotic agent and which narcotic agent is from one of the narcotic agent supply 37 and the gas supply 31. Through the user interface 42 along a signal line 100 the user may set to the controller 40 a target value for a desired narcotic effect of narcotic agents. The desired narcotic effect may be determined in terms of the concentration of the vapor measured in percentage at 101.3 kPa ambient pressure preventing a patient movement under a surgical stimuli of a skin incision in 50% of patients which is often called as a mean alveolar concentration of inhaled narcotic agents (MAC) or the desired narcotic effect may be determined in terms of a concentration suppressing a response on commands in 50% of subjects such as MAC-awake. It is understood the desired narcotic effect value as well as the converted narcotic effect value may be a compensated value in which case some specific matter or matters affecting to the narcosis has been taken into account and possibly compensated. Such specific matter is for example a subject age. Besides the subject age compensated value such as an age MAC also a barometric pressure belongs to compensated values. Also there may be a reason for other compensated values. The barometric pressure can be measured with a pressure sensor (not shown in the figures) or configured using the user interface 42. In this embodiment the user interface 42 is used to inform the controller 40 about the subject age as well.

To take into consideration all narcotic agents of the breathing gas the controller 40 is able to collect from the gas analyzer 39 the information indicative of measured breathing gas concentration of each narcotic agent which can be converted to the narcotic effect value. All narcotic agents should be taken into consideration. These narcotic effect values of each narcotic agent are after a calculation summed up and compared by the controller 40 with the target value received from the user interface 42 and determined whether or not to change the breathing gas concentration. In case the target value deviates from the narcotic effect value converted from measurement results of the narcotic agents the actuator 38 is under the control of the controller 40 adjusting the narcotic agent flow from the narcotic agent supply 37 in order to reduce a deviation. When using $N_2O$ as the balance gas, the subject oxygenation demand determines the narcotic effect available from the $N_2O$. Therefore the flow regulating valve 33 can usually not be used in practice for the narcotic control.

The controller 40 is connected through a signal line 101 to the selector valve 32 of the fresh gas delivery unit 3, which signal line 101 is adapted to carry a signal from the controller 40 to the selector valve 32. Also the controller 40 is connected through a signal line 102 to the flow regulating valve33 of the fresh gas delivery unit, which signal line 102 is adapted to carry a signal from the controller 40 to the flow regulating valve 33. Further the controller 40 is connected through a signal line 103 to the flow sensor 35 of the fresh gas delivery unit 3, which signal line 103 is adapted to carry a signal from this flow sensor 35 to the controller 40. Also the controller 40 is connected through a signal line 104 to the flow regulating valve 34 of the gas delivery unit 3, which signal line 104 is adapted to carry a signal from the controller 40 to the flow regulating valve 34. This controller 40 is also connected through a signal line 105 to the flow sensor 36, which signal line 105 is adapted to carry a signal from the flow sensor 36 to the controller 40. The controller 40 is also connected through a signal line 106 to the gas analyzer 39, which signal line is adapted to carry a signal indicative of a measured alveolar concentration of each narcotic agent from the gas analyzer 39 to the controller 40. The signal line 107 is needed in inhalation anesthesia to carry a signal between the actuator 38 for at least one narcotic agent supply 37 and the controller 40 to adjust the narcotic agent.

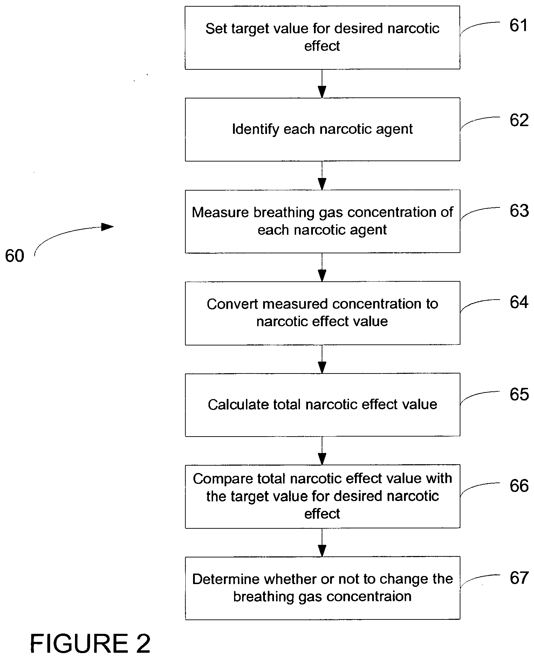

Figure 2 shows a method 60 for controlling the narcotic effect value of a mixture of at least two narcotic agents of the breathing gas. References to the arrangement of Figure 1 have also been made while discussing the method.

At step 61 the target value for the desired narcotic effect is set. The user may do this by means of the user interface 42 and he/she does not necessarily need to consider concentrations of each narcotic agent of the breathing gas, but it is enough to give the target value for desired narcotic effect and select the narcotic agent used and let the controller 40 to take care of the rest. The desired narcotic effect value may be also a compensated value in which case some specific matter or matters affecting to the narcosis has been taken into account and possibly compensated.

In case narcotic agents used in the breathing gas are not known, their identification should be done by means of the gas analyzer 39 at step 62, which is thus optional, but which is in any case advantageous to avoid wrong identifications. This identification information can also be sent via signal line 106 to the controller 40.

Breathing gas concentrations of each narcotic agent at step 63 are measured by the gas analyzer 39. The concentration measurement includes also the nitrous oxide concentration as one possible narcotic agent present in the breathing gas. Typically the concentration of the narcotic agent present in the exhalation gas or especially the end tidal concentration is measured during anesthesia.

The measured breathing gas concentration of each narcotic agent received by the controller 40 is converted to the narcotic effect value at step 64. The controller 40 is also able to do this conversion. The converted narcotic effect value may be also a compensated value in which case some specific matter or matters affecting to the narcosis has been taken into account and possibly compensated. Each narcotic agent has concentration giving determined narcotic effect in 50% of patients. This concentration is assigned as a reference concentration value in calculating the narcotic effect value such as the mean alveolar concentration (MAC), $MAC_{AGE}$ or $MAC_{AWAKE}$. These reference concentration values are often determined at sea-level conditions. At higher altitudes these concentration values are increasing in ratio of barometric pressure decrease. Thus, the narcotic effect of given anesthetic agent at given barometric pressure is calculated as:

$$N = \frac{C \cdot P_{amb}}{C_{ref} \cdot P_{sealevel}},$$

where N is the narcotic effect, C is the alveolar concentration, $C_{ref}$ is reference concentration value providing desired narcotic effect in 50% of patients, $P_{sealevel}$ is the ambient pressure at sea level (101.3 hPa) and $P_{amb}$ is the prevailing ambient pressure at the site of performing the anesthesia. Thus, as an example, having sevoflurane anesthesia at barometric pressure of 95 hPa and measuring patient alveolar concentration of 2.5% represents

$$N = \frac{2.5 \cdot 95}{2.1 \cdot 101.3} = 1.1 MAC_{\mathrm{Pr}essire}.$$

At step 65 when the mixture of at least two narcotic agents have been used a total narcotic effect value of all narcotic agents is calculated by the controller 40. The total narcotic effect value may be a sum of the narcotic effect values of the mixture. The narcotic effect values of the narcotic agents are linear and additive allowing a simple calculation process. So if the narcotic effect value is 0.6 MAC for one agent and 0.5 MAC for another agent then their sum is 1.1 MAC.

The sum of the narcotic effect values is compared at step 66 with the target value set at step 61. Again the controller 40 is able to do this. Based on the comparison the controller 40 can at step 67 determine whether or not to change the breathing gas concentration and may also determine the action needed in order to make the target value and the narcotic effect value to match whereupon the difference between the target value and the narcotic effect value is reduced. If the sum of the narcotic effect value is below the target value, the controller 40 may change or increase at least one narcotic agent's concentration in the breathing gas. This may happen by allowing the actuator 38 to deliver more narcotic agent of the narcotic agent supply 37. On the other hand if the total the narcotic effect value is over the target value the actuator 38 may allow to deliver less narcotic agent from the narcotic agent supply 37. Thus possible actions are increasing, decreasing, or maintaining the delivery of the narcotic agent. The total narcotic effect value is allowed deviate from the target value less than 30%, more specifically less than 20% or, even more specifically less than 10% without changing the narcotic agent concentration in the breathing gas. This deviation can be allowed to both directions. Using this method or arrangement of the embodiment the controller 40 automatically responds in changing the delivery of the narcotic agent when the concentrations of the other narcotic agents are changing. E.g. when changing from one agent to another during anesthesia involves gradual increment of the new narcotic agent concentration corresponding to the clearance of the old narcotic agent from the body.

This embodiment provides ease of use during changes in breathing gas O2 concentration and in changing between different inhalation narcotic agents during the anesthesia. The embodiment also provides a patient safety in preventing accidental over- and under-delivery of the narcotic agent during these transient phases.

The written description uses examples to disclose the invention, including the best mode, and also to enable any person skilled in the art to make and use the invention. The patentable scope of the invention is defined by the claims, and may include other examples that occur to those skilled in the art.

## Claims

1. An arrangement for controlling a narcotic effect value of a mixture of at least two narcotic agents of a breathing gas, the arrangement comprising:

   a gas delivery unit (3) to be connected to a breathing circuit (2) for supplying a breathing gas including a mixture of at least two narcotic agents for a respiration, said gas delivery unit comprising at least one narcotic agent supply (37);
   a gas analyzer (39) for measuring an exhalation gas concentration of each narcotic agent of the breathing gas; and
   a user interface (42) for setting a target value for a desired narcotic effect of narcotic agents, **characterized in that** the arrangement also comprising a controller (40) for converting the measured concentration of each narcotic agent to a narcotic effect value of the breathing gas, for calculating a total narcotic effect value of the mixture of said at least two narcotic agents, for comparing the total narcotic effect value with the target value and for determining whether or not to change said breathing gas concentration to meet said target value.

2. The arrangement according to claim 1, further comprising a ventilator (1) configured to control respiratory movements and a breathing circuit (2) for conducting an expiration gas flow to said ventilator (1) and for conducting the fresh gas flow from said gas delivery unit (3) for the respiration and for conducting the ventilator gas flow for the inspiration.

3. The arrangement according to claim 1, **characterized in that** a controller (40) is configured to receive from said user interface (42) the target value for the desired narcotic effect which is compensated value as well as the converted narcotic effect value in which case some specific matter or matters affecting to the narcosis has been taken into account.

4. The arrangement according to claim 1, **character-**

**ized in that** said a gas analyzer (39) is configured to identify narcotic agents.

5. The arrangement according to claim 1, **characterized in that** said gas delivery unit (3) includes a flow regulating valve (33) to regulate a balance gas including the narcotic agent.

6. The arrangement according to claim 1, **characterized in that** said gas delivery unit (3) includes an actuator (38) to adjust the narcotic agent vaporized by means of the narcotic agent supply (37).

7. The arrangement according to claim 1, **characterized in that** said target value for the desired narcotic effect as well as the converted narcotic effect value is a compensated value in which case some specific matter or matters affecting to the narcosis has been taken into account.

8. The arrangement according to claim 1, **characterized in that** the total narcotic effect value is allowed to deviate from the target value less than 30%, more specifically less than 20% or, even more specifically less than 10% without changing the narcotic agent concentration in the breathing gas.

## Patentansprüche

1. Anordnung zur Steuerung eines Narkoseauswirkungswerts eines Gemischs von mindestens zwei Narkosemitteln eines Atemgases, wobei die Anordnung das Folgende umfasst:

   eine Gaszuführungseinheit (3) zur Verbindung mit einem Atemkreislauf (2) zum Zuführen eines Atemgases mit einem Gemisch von mindestens zwei Narkosemitteln für eine Atmung, wobei die Gaszuführungseinheit mindestens eine Narkosemittelversorgung (37) umfasst;
   einen Gasanalysator (39) zum Messen einer Ausatemgaskonzentration jedes Narkosemittels des Atemgases; und
   eine Benutzeroberfläche (42) zum Einstellen eines Zielwerts für eine gewünschte Narkoseauswirkung von Narkosemitteln, **dadurch gekennzeichnet, dass** die Anordnung auch eine Steuereinheit (40) zum Umrechnen der gemessenen Konzentration jedes Narkosemittels in einen Narkoseauswirkungswert des Atemgases, zum Berechnen eines Narkoseauswirkungsgesamtwerts des Gemisches der mindestens zwei Narkosemittel, zum Vergleichen des Narkoseauswirkungsgesamtwerts mit dem Zielwert und zum Bestimmen, ob die Atemgaskonzentration zum Erreichen des Zielwerts geändert werden muss oder nicht, umfasst.

**2.** Anordnung nach Anspruch 1, ferner umfassend ein Beatmungsgerät (1), das zur Steuerung der Atembewegungen ausgelegt ist, und einen Atemkreislauf (2) zum Leiten einer Ausatemgasströmung zum Beatmungsgerät (1) und zum Leiten der frischen Gasströmung von der Gaszuführungseinheit (3) für die Atmung und zum Leiten der Beatmungsgasströmung für die Einatmung.

**3.** Anordnung nach Anspruch 1, **dadurch gekennzeichnet, dass** eine Steuereinheit (40) dazu ausgelegt ist, von der Benutzeroberfläche (42) den Zielwert für die gewünschte Narkoseauswirkung, der ein kompensierter Wert ist, und den umgerechneten Narkoseauswirkungswert zu empfangen, in welchem Fall ein bestimmter Aspekt oder bestimmte Aspekte, der bzw. die einen Einfluss auf die Narkose haben, berücksichtigt wurden.

**4.** Anordnung nach Anspruch 1, **dadurch gekennzeichnet, dass** ein Gasanalysator (39) zur Identifizierung von Narkosemitteln ausgelegt ist.

**5.** Anordnung nach Anspruch 1, **dadurch gekennzeichnet, dass** die Gaszuführungseinheit (3) ein Durchflussregelventil (33) zur Regulierung eines Gleichgewichtsgases, einschließlich des Narkosemittels aufweist.

**6.** Anordnung nach Anspruch 1, **dadurch gekennzeichnet, dass** die Gaszuführungseinheit (3) ein Betätigungselement (38) zum Einstellen des mittels der Narkosemittelversorgung (37) verdampften Narkosemittels aufweist.

**7.** Anordnung nach Anspruch 1, **dadurch gekennzeichnet, dass** der Zielwert für die gewünschte Narkoseauswirkung sowie der umgerechnete Narkoseauswirkungswert ein kompensierter Wert ist, in welchem Fall ein bestimmter Aspekt oder bestimmte Aspekte, der bzw. die einen Einfluss auf die Narkose haben, berücksichtigt wurden.

**8.** Anordnung nach Anspruch 1, **dadurch gekennzeichnet, dass** der Narkoseauswirkungsgesamtwert von Zielwert um weniger als 30%, insbesondere weniger als 20%, besonders weniger als 10% abweichen darf, ohne die Narkosemittelkonzentration im Atemgas zu verändern.

**Revendications**

**1.** Agencement permettant de contrôler une valeur d'effet narcotique d'un mélange d'au moins deux agents narcotiques d'un gaz respiratoire, l'agencement comprenant :

une unité d'administration (3) de gaz devant être raccordée à un circuit respiratoire (2) pour fournir un gaz respiratoire comprenant un mélange d'au moins deux agents narcotiques pour une respiration, ladite unité d'administration de gaz comprenant au moins une alimentation (37) en agent narcotique ;
un analyseur (39) de gaz permettant de mesurer une concentration dans le gaz d'expiration de chaque agent narcotique du gaz respiratoire ; et
une interface utilisateur (42) pour définir une valeur cible pour un effet narcotique souhaité d'agents narcotiques,
**caractérisé en ce que** l'agencement comprend également un organe de commande (40) pour convertir la concentration mesurée de chaque agent narcotique en une valeur d'effet narcotique du gaz respiratoire, pour calculer une valeur d'effet narcotique totale du mélange desdits au moins deux agents narcotiques, pour comparer la valeur d'effet narcotique totale avec la valeur cible et pour déterminer si oui ou non il convient de changer ladite concentration de gaz respiratoire pour atteindre ladite valeur cible.

**2.** Agencement selon la revendication 1, comprenant en outre un ventilateur (1) conçu pour contrôler les mouvements respiratoires et un circuit de respiration (2) pour conduire un flux de gaz d'expiration vers ledit ventilateur (1) et pour conduire le flux de gaz frais depuis ladite unité d'administration (3) de gaz pour la respiration et pour conduire le flux de gaz de ventilateur pour l'inspiration.

**3.** Agencement selon la revendication 1, **caractérisé en ce qu'**un organe de commande (40) est conçu pour recevoir, de ladite interface utilisateur (42), la valeur cible pour l'effet narcotique souhaité qui est une valeur compensée ainsi que la valeur d'effet narcotique convertie, auquel cas une ou plusieurs matières spécifiques affectant la narcose ont été prises en compte.

**4.** Agencement selon la revendication 1, **caractérisé en ce que** ledit analyseur (39) de gaz est conçu pour identifier des agents narcotiques.

**5.** Agencement selon la revendication 1, **caractérisé en ce que** ladite unité d'administration (3) de gaz comprend une valve de régulation de flux (33) pour réguler un gaz de complément comprenant l'agent narcotique.

**6.** Agencement selon la revendication 1, **caractérisé en ce que** ladite unité d'administration (3) de gaz comprend un actionneur (38) pour ajuster l'agent narcotique vaporisé au moyen de l'alimentation (37) en agent narcotique.

7. Agencement selon la revendication 1, **caractérisé en ce que** ladite valeur cible pour l'effet narcotique souhaité ainsi que la valeur d'effet narcotique convertie sont une valeur compensée, auquel cas une ou plusieurs matières spécifiques affectant la narcose ont été prises en compte.

8. Agencement selon la revendication 1, **caractérisé en ce que** la valeur d'effet narcotique totale peut s'écarter de la valeur cible de moins de 30 %, plus particulièrement de moins de 20 %, ou encore plus particulièrement de moins de 10 % sans changer la concentration en agent narcotique dans le gaz respiratoire.

FIGURE 1

EP 2 316 515 B1

```
                          ┌──────────────────────────────┐
                          │  Set target value for desired │ ⌐─ 61
                          │      narcotic effect          │
                          └──────────────────────────────┘
                                        │
                                        ▼
                          ┌──────────────────────────────┐
                          │    Identify each narcotic     │ ⌐─ 62
                          │           agent               │
                          └──────────────────────────────┘
                                        │
                                        ▼
                          ┌──────────────────────────────┐
                          │ Measure breathing gas         │ ⌐─ 63
                          │ concentration of each         │
                          │ narcotic agent                │
                          └──────────────────────────────┘
                                        │
                                        ▼
                          ┌──────────────────────────────┐
                          │ Convert measured concentration│ ⌐─ 64
                          │ to narcotic effect value      │
                          └──────────────────────────────┘
                                        │
                                        ▼
                          ┌──────────────────────────────┐
                          │ Calculate total narcotic      │ ⌐─ 65
                          │ effect value                  │
                          └──────────────────────────────┘
                                        │
                                        ▼
                          ┌──────────────────────────────┐
                          │ Compare total narcotic effect │ ⌐─ 66
                          │ value with the target value   │
                          │ for desired narcotic effect   │
                          └──────────────────────────────┘
                                        │
                                        ▼
                          ┌──────────────────────────────┐
                          │ Determine whether or not to   │ ⌐─ 67
                          │ change the breathing gas      │
                          │ concentraion                  │
                          └──────────────────────────────┘
```

60

## FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006094172 A

**Non-patent literature cited in the description**

- **EDMOND I ; EGER II.** Age, Minimum Alveolar Anesthetic Concentration, and Minimum Alveolar Anesthetic Concentration-Awake. *Anesth Analg.,* 2001, vol. 93, 947-53